# EUROPÄISCHE PATENTSCHRIFT

(11) **EP 3 428 030 B1**
(45) Veröffentlichungstag und Bekanntmachung des Hinweises auf die Patenterteilung: **10.01.2024**
(21) Anmeldenummer: 18174867.4
(22) Anmeldetag: 29.05.2018
(51) Int. Cl.: B60W 50/00, B60W 40/09, B60W 40/08

(54) **VERFAHREN ZUM ANPASSEN ZUMINDEST EINES BETRIEBSPARAMETERS EINES KRAFTFAHRZEUGS, SYSTEM ZUM ANPASSEN ZUMINDEST EINES BETRIEBSPARAMETERS EINES KRAFTFAHRZEUGS UND KRAFTFAHRZEUG**
METHOD FOR ADAPTING AT LEAST ONE OPERATING PARAMETER OF A MOTOR VEHICLE, SYSTEM FOR ADAPTING AT LEAST ONE OPERATING PARAMETER OF A MOTOR VEHICLE AND MOTOR VEHICLE
PROCÉDÉ DE RÉGLAGE D'AU MOINS UN PARAMÈTRE DE FONCTIONNEMENT D'UN VÉHICULE AUTOMOBILE, SYSTÈME DE RÉGLAGE D'AU MOINS UN PARAMÈTRE DE FONCTIONNEMENT D'UN VÉHICULE AUTOMOBILE ET VÉHICULE AUTOMOBILE

(30) Priorität: 12.07.2017 DE 102017211931
(43) Veröffentlichungstag der Anmeldung: 16.01.2019
(73) Patentinhaber: VOLKSWAGEN AKTIENGESELLSCHAFT, 38440 Wolfsburg (DE)
(72) Erfinder: Ehmann, Sebastian, 38302 Wolfenbüttel (DE)

(56) Entgegenhaltungen:
- WO-A1-2016/047063
- DE-A1-102013 210 941
- DE-A1-102013 223 684
- DE-A1-102015 008 150
- DE-A1-102015 203 354
- JP-A- 2016 052 881
- US-A1- 2016 039 424

## Beschreibung

Die Erfindung betrifft ein Verfahren zum Anpassen zumindest eines Betriebsparameters eines Kraftfahrzeugs. Die Erfindung betrifft auch ein System zum Anpassen zumindest eines Betriebsparameters eines Kraftfahrzeugs und ein Kraftfahrzeug mit einem derartigen System.

Die Ausprägung und Gestaltung von Fahrerassistenzfunktionen, welche durch ein oder mehrere Fahrerassistenzsysteme eines Kraftfahrzeugs gesteuert werden, hängt stark von dem subjektiven Empfinden der zu erwartenden Passagiere, also Fahrzeuginsassen, im Kraftfahrzeug ab. Beispielsweise kann bei einem Abstands-Regel-Tempomat als Fahrerassistenzsystem, der die Geschwindigkeit des Kraftfahrzeugs regelt und dabei berücksichtigt, ob sich andere Kraftfahrzeuge vor dem eigenen befinden, eingestellt werden, ab welchem Minimalabstand zu einem vorausfahrenden Kraftfahrzeug ein Bremsvorgang eingeleitet wird. Hierbei kommt es auf das Empfinden des jeweiligen Fahrzeuginsassen an, ob der Beginn des Bremsvorgangs als zu früh oder zu spät, beziehungsweise die Verzögerung als zu schwach oder zu stark empfunden wird. Ein weiteres Beispiel ist die Querbeschleunigung, die bei einer von einem Fahrerassistenzsystem, zum Beispiel einem Spurhalteassistenten, durchgeführten Kurvenfahrt auftritt. Eine Querbeschleunigung und die damit verbundene Durchfahrtsgeschwindigkeit durch eine Kurve, die von dem Fahrzeuginsassen noch akzeptiert wird, können für einen anderen Fahrzeuginsassen schon als unangenehm empfunden werden.

Aus dem allgemeinen Stand der Technik ist es bekannt, dass bei einem Fahrerassistenzsystem ein Parameter von dem Fahrzeuginsassen, wie beispielsweise dem Fahrer, eingestellt oder von dem Fahrzeuginsassen eingelernt werden kann. Hierzu beschreibt beispielsweise die DE 10 2014 118 079 A1 ein Kraftfahrzeug, welches einen autonomen Fahrsensor umfasst, welcher dazu eingerichtet ist, wenigstens einen Zustand zu erfassen, während das Fahrzeug in einer autonomen Betriebsweise betrieben wird. Eine Verarbeitungsvorrichtung des Kraftfahrzeugs ist dazu eingerichtet, wenigstens ein Fahrzeugteilsystem zu steuern, während das Fahrzeug in der autonomen Betriebsweise betrieben wird. Die Verarbeitungsvorrichtung ist ferner dazu eingerichtet, dass wenigstens eine Fahrzeugteilsystem gemäß einer Fahrerbevorzugung zu steuern.

Anstelle für ein Fahrerassistenzsystem Parameter zu definieren kann das Kraftfahrzeug selbst dazu eingerichtet sein, vorausschauend zu agieren. Hierzu beschreibt die EP 2 862 773 A2 ein Kraftfahrzeug, welches ein Fahrerassistenzsystem zur Vorausberechnung von Voraussagedaten über wenigstens eine zukünftige Fahrsituation des Kraftfahrzeugs durch Auswertung von das Kraftfahrzeug betreffenden Egodaten und das Kraftfahrzeugumfeld betreffenden Umfelddaten umfasst. Das Kraftfahrzeug ist in einem ersten Betriebsmodus des Fahrerassistenzsystems durch einen Fahrer steuerbar, wobei das Fahrerassistenzsystem dazu ausgebildet ist, bei Erfüllung einer Auslösebedingung, temporär in einen zweiten Betriebsmodus umzuschalten, in dem die Steuerung des Kraftfahrzeugs ohne Eingriffsmöglichkeiten durch den Fahrer autonom durch das Fahrerassistenzsystem erfolgt. Die Auslösebedingung ist dazu ausgebildet, zumindest die Voraussagedaten und wenigstens eine Fahrereigenschaft beschreibende Fahrereigenschaftsdaten auszuwerten.

Der Nachteil derartiger Fahrerassistenzsysteme besteht darin, dass wenn sich der Fahrer oder Mitfahrer des Kraftfahrzeugs aufgrund der voreingestellte Parameter nicht wohl fühlen sollte das Kraftfahrzeug entweder in einem manuellen Fahrbetrieb zu betreiben ist oder die Parameter durch den Fahrer weiterhin selbst anzupassen sind. Bei einer zunehmenden Anzahl von Fahrerassistenzsystemen wird es für den Fahrzeuginsassen immer umständlicher, alle Parameter selbst zu definieren. Auch können für den Fahrzeuginsassen akzeptable Werte der Parameter im Tagesverlauf oder in Abhängigkeit von einem persönlichen Zustand des Fahrzeuginsassen schwanken. Ebenfalls können die Parameterbereiche in Abhängigkeit von der Anzahl und Art der Fahrzeuginsassen schwanken.

In der DE 10 2013 210 941 A1 ist ein Verfahren zum Betreiben eines Fahrzeugs beschrieben, bei dem eine Fahrzeugführung eines Fahrers des Fahrzeugs überwacht wird, sodass basierend auf der überwachten Fahrzeugführung einen Fahrstil des Fahrers beschreibende Fahrparameter gebildet werden. Diese Fahrparameter werden einem Fahrerassistenzsystem des Fahrzeugs bereitgestellt, sodass bei einer Steuerung des Fahrzeugs mittels des Fahrerassistenzsystems der Fahrstil des Fahrers abgebildet wird. Wenn der Fahrer das Fahrzeug bei einer automatisierten Fahrt abbremst, so ist dies in der Regel ein Hinweis darauf, dass ein Abstand zu dem unmittelbar vorausfahrenden Fahrzeug zu niedrig für den Fahrer ist und/oder dass das Fahrerassistenzsystem zu schnell für den Fahrer ist, also mit einer zu hohen Geschwindigkeit das Fahrzeug steuert. Entsprechend wird der Fahrstil angepasst.

In der JP 2016 052 881 A ist ein Verfahren zum Betreiben einer Fahrzeugfahrsteuerung beschrieben. Bei dem Verfahren wird ein Zusammenhang zwischen einem Fahrzustand des Fahrzeugs und einem Unwohlsein des Fahrers bestimmt. Hierzu wird während dem Betrieb des Kraftfahrzeugs der Fahrer überwacht. Basierend auf dem bestimmten Unwohlfaktor passt eine Fahrsteuerungsvorrichtung einen Steuergrad der Fahrzeugfahrsteuerung an und ändert die Fahrzeugfahrsteuerung, um das Unbehagen des Fahrers zu verringern.

In der DE 10 2015 203 354 A1 ein System für ein Fahrzeug beschrieben. Das System umfasst einen Computer, der einen Prozessor und einen Speicher umfasst. Der Computer ist dazu konfiguriert, einen oder mehrerer Betriebsabläufe des Fahrzeugs ohne Insasseneingabe auszuführen, Daten, die einen Insassenbasiszustand betreffen, zu speichern, Daten, die einen gegenwärtigen Insassenzustand betreffen zu sammeln, einen Vergleich des Insassenbasiszustands mit dem gegenwärtigen Insassenzustand auszuführen und einen Parameter, der die Ausführung des einen oder der mehreren Betriebsabläufe in Übereinstimmung mit dem Vergleich regelt, auszuführen.

In der US 2016/0039424 A1 trägt ein Fahrer eine Armbanduhr mit Display. Mit Hilfe der Armbanduhr wird ein Zustand des Fahrers erfasst, zum Beispiel ob der Fahrer sich in einem müden oder aufgeregten Zustand befindet. Sollte ein kritischer Zustand (Müdigkeit, Aufgeregtheit etc.) des Fahrers erfasst werden, welches gefährlich für eine Fahrt mit dem Kraftfahrzeug sein könnte, so wird dem Fahrer auf dem Display der Armbanduhr eine Anzeige geschaltet, welche den Zustand des Fahrers überprüft. Stimmt der Fahrer zu, also bestätigt der Fahrer den Zustand, so wird eine Empfehlung ausgegeben, das Fahrzeug gerade nicht zu fahren.

Aufgabe der vorliegenden Erfindung ist es daher, ein Verfahren zum Anpassen zumindest eines Betriebsparameters eines Kraftfahrzeugs sowie ein System zum Anpassen zumindest eines Betriebsparameters des Kraftfahrzeugs bereitzustellen, bei welchem der zumindest eine Betriebsparameter auf besonders einfache Art und Weise anpassbar und damit eine Zuverlässigkeit, insbesondere hinsichtlich einer Kompatibilität des angepassten Betriebsparameters für einen Fahrer oder Mitfahrer des Kraftfahrzeugs, erhöht wird.

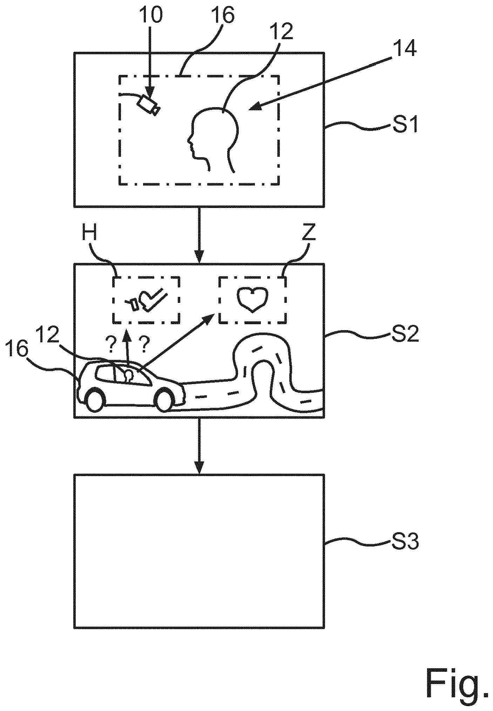

Die Aufgabe wird durch die Gegenstände der unabhängigen Patentansprüche gelöst. Vorteilhafte Weiterbildungen der Erfindung sind durch die Merkmale der abhängigen Patentansprüche, die folgende Beschreibung sowie die Figur offenbart.

Erfindungsgemäß wird diese Aufgabe gelöst durch ein Verfahren zum Anpassen zumindest eines Betriebsparameters eines Kraftfahrzeugs während eines Fahrmanövers des Kraftfahrzeugs, wobei der zumindest eine Betriebsparameter des Kraftfahrzeugs durch zumindest ein Fahrerassistenzsystem des Kraftfahrzeugs eingestellt wird. Mit "Betriebsparameter" ist bevorzugt ein Fahrprofil oder ein Fahrzustand oder eine Fahreigenschaft des Kraftfahrzeugs beschreibender Parameter, also ein Fahrparameter, gemeint. Bevorzugt wird als der zumindest eine Betriebsparameter eine Geschwindigkeit des Kraftfahrzeugs und/oder eine Beschleunigung, insbesondere eine Querbeschleunigung des Kraftfahrzeugs und/oder eine Längsbeschleunigung des Kraftfahrzeugs, und/oder ein Abstand zu einem vorausfahrenden Kraftfahrzeug und/oder ein Halten einer Fahrspur des Kraftfahrzeugs und/oder ein Lenkmoment angepasst. Mit "Anpassen des zumindest einen Betriebsparameters" ist insbesondere ein Ändern oder ein Einstellen oder ein Wechsel eines vorbestimmten oder voreingestellten Betriebsparameters zu einem neuen Betriebsparameter gemeint. Mit "Fahrmanöver" ist insbesondere eine Aktion oder eine Durchführung eines Manövers des Kraftfahrzeugs gemeint. Beispielsweise kann es sich bei dem Fahrmanöver um ein Fahren des Kraftfahrzeugs und/oder ein Überholen und/oder einen Spurwechsel und/oder ein Abbiegen und/oder ein Rückwärtsfahren und/oder ein Anhalten und/oder ein Anfahren und/oder ein Beschleunigen und/oder eine Kurvenfahrt und/oder ein Einparken und/oder ein Ausparken handeln. Insbesondere definiert oder beeinflusst der eingestellte Betriebsparameter das Fahrmanöver. Beispielsweise kann ein Fahrerassistenzsystem dazu eingerichtet sein, eine Kurvenfahrt des Kraftfahrzeugs zu überwachen und/oder zu steuern. Hierzu kann dem Fahrerassistenzsystem zur Durchführung der Kurvenfahrt als Fahrmanöver eine Querbeschleunigung als Betriebsparameter vorgegeben sein.

Bei dem Verfahren wird zunächst wenigstens ein Fahrzeuginsasse identifiziert, wobei bei der Identifizierung des Fahrzeuginsassen biometrische Merkmale erfasst werden. Anschließend wird ein vorbestimmtes Fahrprofil für den Fahrzeuginsassen erstellt oder geladen.

Bei dem Verfahren wird in einem weiteren Verfahrensschritt zumindest eine Aktivität eines Fahrzeuginsassen während des Fahrmanövers in Reaktion auf das Fahrmanöver mittels einer Erfassungseinrichtung erfasst. Hierzu kann die Erfassungseinrichtung dazu eingerichtet sein, ein aktuelles Fahrmanöver des Kraftfahrzeugs zu erfassen. Alternativ kann auch das zumindest eine Fahrerassistenzsystem das Fahrmanöver signalisieren, woraufhin eine Steuereinrichtung die Erfassungseinrichtung ansteuern kann. Während des erfassten Fahrmanövers wird der Fahrzeuginsasse bevorzugt überwacht oder beobachtet. Mit anderen Worten kann die Erfassungseinrichtung dazu eingerichtet sein, eine Veränderung einer Verhaltens und/oder Zustands des Fahrzeuginsassen während des Fahrmanövers zu erfassen. Bei dem Fahrzeuginsassen kann es sich beispielsweise um einen Fahrer oder einen Mitfahrer des Kraftfahrzeugs handeln. Alternativ oder zusätzlich kann die Erfassungseinrichtung dazu eingerichtet sein, zumindest einen weiteren Fahrzeuginsassen zu erfassen und zumindest eine Aktivität des weiteren Fahrzeuginsassen während des Fahrmanövers in Reaktion auf das Fahrmanöver mittels der Erfassungseinrichtung zu erfassen.

In einem weiteren Verfahrensschritt wird der zumindest eine Betriebsparameter des Kraftfahrzeugs in Abhängigkeit von der erfassten Aktivität des Fahrzeuginsassen durch Ansteuern des zumindest einen Fahrerassistenzsystems mittels einer Steuereinrichtung angepasst oder eingestellt. Hierzu kann ein Wert des zumindest einen Betriebsparameters des Kraftfahrzeugs, welcher das aktuelle Fahrmanöver beeinflusst oder mit welchem das aktuelle Fahrmanöver durchgeführt wird, geändert werden. Zusätzlich kann es vorgesehen sein, dass der zumindest eine Betriebsparameter in Abhängigkeit von der erfassten Aktivität des Fahrzeuginsassen und in Abhängigkeit von dem aktuellen Fahrmanöver angepasst wird. Beim Anpassen des zumindest einen Betriebsparameters kann die Steuereinrichtung dazu eingerichtet sein, einen Wert des Betriebsparameters zu erhöhen oder zu reduzieren. Damit der zumindest eine Betriebsparameter durch die Steuereinrichtung zuverlässig auf die Reaktion des zumindest einen Fahrzeuginsassen hin angepasst werden kann, können mehrere Aktivitäten in einem Speicher der Steuereinrichtung hinterlegt sein. Je nachdem welche Aktivität erfasst wird, kann die Steuereinrichtung dazu eingerichtet sein, den aktuell eingestellten Wert des zumindest einen Betriebsparameters zu erhöhen oder zu reduzieren, also hochzusetzen oder runterzusetzen. Wird insbesondere keine Aktivität oder keine Veränderung der Aktivität des zumindest einen Fahrzeuginsassen durch die Erfassungseinrichtung erfasst, so wird der zumindest eine Betriebsparameter bevorzugt nicht angepasst. Mit anderen Worten kann, wenn keine Aktivität oder keine Veränderung der Aktivität des zumindest einen Fahrzeuginsassen durch die Erfassungseinrichtung erfasst wird, der aktuelle eingestellte Betriebsparameter beibehalten werden.

Einem Fahrmanöver können auch mehrere Betriebsparameter zugewiesen sein. Beispielsweise können bei einer Kurvenfahrt als Fahrmanöver ein Lenkmoment und eine Querbeschleunigung vorgegeben sein. Entsprechen können zusätzlich oder alternativ auch mehrere Betriebsparameter durch die Steuereinrichtung in Abhängigkeit von dem Fahrmanöver angepasst werden.

Durch das Verfahren wird eine direkte Kopplung zwischen dem Betriebsparameter zu einem Fahrbetrieb des Kraftfahrzeugs hergestellt. Dabei kann der zumindest eine Betriebsparameter direkt in Reaktion auf die Aktivität des Fahrzeuginsassen hin angepasst werden, da die Reaktion durch das Fahrmanöver hervorgerufen wurde. Durch das Verfahren kann auf die Bedürfnisse oder das Verhalten oder die Aktivität des Fahrzeuginsassen direkt, besonders schnell und zuverlässig eingegangen werden.

Erfindungsgemäß ist es vorgesehen, dass zum Anpassen des zumindest einen Betriebsparameters des Kraftfahrzeugs eine Bestätigungshandlung auf eine Zustandsanfrage hinsichtlich der Reaktion des Fahrzeuginsassen auf das Fahrmanöver, welche durch eine Ausgabeeinrichtung ausgegeben wird, erfasst wird. Beispielsweise kann es sich bei der Ausgabeeinrichtung einen Bildschirm, insbesondere zur schriftlichen Ausgabe der Zustandsanfrage, und/oder um einen Lautsprecher, insbesondere zur akustischen Ausgabe der Zustandsanfrage, handeln. Bevorzugt ist die Ausgabeeinrichtung in dem Innenraum des Kraftfahrzeugs angeordnet. Handelt es sich bei der Ausgabeeinrichtung beispielsweise um einen Bildschirm, so kann dieser in einem Sichtfeld des Fahrers, beispielsweise an einer Mittelkonsole oder an einem Armaturenbrett des Kraftfahrzeugs, angeordnet sein. Insbesondere kann die Ausgabeeinrichtung dazu eingerichtet sein, sich durch Ansteuern einer Steuereinrichtung, nach dem Befinden des Fahrzeuginsassen zu erkundigen. Beispielsweise kann die Ausgabeeinrichtung dazu eingerichtet sein, schriftlich und/oder akustisch eine Wortlaut, insbesondere eine Frage, welche den Zustand des Fahrzeuginsassen betrifft, auszugeben. Beispielsweise kann als Wortlaut "Ist das Kraftfahrzeug geradezu knapp eingeschert oder abgebogen? oder "Ist das Kraftfahrzeug gerade schnell durch die Kurve gefahren?" oder "Ist alles in Ordnung?" ausgegeben werden. Daraufhin kann die Erfassungseinrichtung dazu eingerichtet sein, eine Bestätigungshandlung oder eine Zustimmungshandlung des Fahrzeuginsassen auf die Zustandsanfrage hin zu erfassen. Beispielsweise kann die Erfassungseinrichtung dazu eingerichtet sein, ein Nicken oder Kopfschütteln des Fahrzeuginsassen oder eine Bedienhandlung an einem Bedienelement oder eine akustische Äußerung, wie zum Beispiel "ja" oder "nein", zu erfassen. Dadurch kann sichergestellt werden, dass die Betriebsparameter nicht versehentlich, also ohne eine Zustimmung des Fahrzeuginsassen, angepasst werden. Zudem erhält der Fahrzeuginsasse aufgrund seiner Reaktion eine direkte Rückmeldung, wodurch der Fahrzeuginsasse feststellen kann, dass seine Reaktion wahrgenommen wurde.

Erfindungsgemäß ist es vorgesehen, dass als die Aktivität des Fahrzeuginsassen eine Schutzhandlung des Fahrzeuginsassen zum Personenschutz des Fahrzeuginsassen erfasst wird. Mit anderen Worten kann als die Aktivität des Fahrzeuginsassen durch die Erfassungseinrichtung erfasst werden, ob der Fahrzeuginsasse in Reaktion auf das Fahrmanöver hin eine Schutzhaltung einnimmt. Als Schutzhandlung wird eine akustische Äußerung des Fahrzeuginsassen und/oder ein Festhalten und/oder ein Abstützen des Fahrzeuginsassen an einem Innenausstattungselement des Kraftfahrzeugs, insbesondere an einem Griff in einem Innenraum des Kraftfahrzeugs und/oder an einer Sitzlehne eines Sitzes und/oder einer Armlehne des Sitzes und/oder einer Tür des Kraftfahrzeugs, erfasst. Als akustische Äußerung des Fahrzeuginsassen kann die Erfassungseinrichtung beispielsweise eine Aussage oder einen Befehl oder ein Geräusch des Fahrzeuginsassen erfassen. Hierzu kann die Erfassungseinrichtung eine Spracherkennungseinrichtung, wie beispielsweise ein Mikrofon, aufweisen. Als Aussage oder Befehl kann der Fahrzeuginsasse beispielsweise als Wortlaut "das war aber knapp" oder "nicht so drängeln" oder "etwas langsamer" ausgeben oder aussprechen. Als Geräusch kann die Erfassungseinrichtung beispielsweise dazu eingerichtet sein, ein Seufzen oder Laute, die auf ein Erschrecken hindeuten oder hinweisen, oder ein Stöhnen erfassen. Beispielsweise können zum Erfassen der akustischen Äußerungen des Fahrzeuginsassen in einem Speicher der Erfassungseinrichtung akustische Daten der Geräusche und/oder der Wortlaute und/oder der Aussagen und/oder der Befehle hinterlegt sein. Eine Steuereinrichtung der Erfassungseinrichtung kann dazu eingerichtet sein, die erfassten akustischen Äußerungen mit den gespeicherten akustischen Daten zu vergleichen. Erfasst beispielsweise die Erfassungseinrichtung das Wort "langsamer", so kann die Steuereinrichtung dazu eingerichtet sein, den aktuellen Wert des zumindest einen Betriebsparameters zu reduzieren. Zum Erfassen des Festhaltens des Fahrzeuginsassen und/oder des Abstützens des Fahrzeuginsassen an einem Innenausstattungselement, wie beispielsweise einen Sitz oder einem Griff des Kraftfahrzeugs, kann die Erfassungseinrichtung beispielsweise einen Sensor, insbesondere einen kapazitiven Sensor, welcher an dem zumindest einen Innenausstattungselements angeordnet ist, aufweisen.

Die Erfassungseinrichtung kann also dazu eingerichtet sein, bewusstes und/oder unbewusstes Verhalten des Fahrzeuginsassen in Reaktion auf das aktuelle Fahrmanöver zu analysieren und auswerten oder erfassen, also wie der Fahrzeuginsasse auf das aktuelle Fahrmanöver reagiert. Damit kann besonders zuverlässig bestimmt werden, für welche der Fahrerassistenzsysteme welche Parameterbereiche für den Fahrzeuginsassen als angenehm oder unangenehm empfunden und/oder eingestuft werden.

Die Erfindung umfasst auch Weiterbildungen des erfindungsgemäßen Verfahrens durch die sich zusätzliche Vorteile ergeben.

Eine vorteilhafte Ausführungsform sieht vor, dass als die Aktivität des Fahrzeuginsassen eine Herzfrequenz und/oder ein Puls und/oder eine Atemfrequenz und/oder eine Gehirnaktivität und/oder ein Müdigkeitszustand des Fahrzeuginsassen erfasst wird. Mit anderen Worten wird ein Zustandsparameter des Fahrzeuginsassen ermittelt. Zur Erfassung der Herzfrequenz des Fahrzeuginsassen kann die Erfassungseinrichtung beispielsweise ein Pulsmessgerät und/oder ein Fitnessarmband umfassen. Zur Erfassung der Atemfrequenz des Fahrzeuginsassen und/oder des Müdigkeitszustands des Fahrzeuginsassen kann die Erfassungseinrichtung beispielsweise eine Kamera aufweisen. Anhand von Bilddaten der Kamera kann zur Erfassung der Atemfrequenz beispielsweise analysiert werden, in welcher Frequenz, also wie oft, sich ein Brustkorb des Fahrzeuginsassen hebt und senkt. Zusätzlich oder alternativ kann anhand der Bilddaten der Kamera eine Blinzelfrequenz von Augen des Fahrzeuginsassen zur Erfassung des Müdigkeitszustands erfasst werden. Beispielsweise kann als Müdigkeitszustand erfasst werden, ob der Fahrzeuginsasse schläft oder nicht. Zur Erfassung der Gehirnaktivität des Fahrzeuginsassen kann die Erfassungseinrichtung beispielsweise ein Elektro-Enzephalogramm (kurz EEG) aufweisen. Durch derartige Vorrichtungen zur Erfassung des Zustandsparameters kann auf besonders einfache und zuverlässige Art und Weise auf die Aktivität des Fahrzeuginsassen geschlossen werden. Wird beispielsweise während einer Kurvenfahrt des Kraftfahrzeugs ein Puls des Fahrzeuginsassen erfasst und liegt der Puls oberhalb eines vorbestimmten Grenzwertes oder Schwellwertes, so kann die Steuereinrichtung dazu eingerichtet sein, das Fahrerassistenzsystem derart anzusteuern, dass die Querbeschleunigung reduziert wird. Wird beispielsweise während eines Bremsvorganges des Kraftfahrzeugs der Puls des Fahrzeuginsassen erfasst und liegt der Puls oberhalb des vorbestimmten Schwellwertes, so kann die Steuereinrichtung dazu eingerichtet sein, das Fahrerassistenzsystem derart anzusteuern, dass ein Abstand zu einem vorausfahrenden Kraftfahrzeug, bei dem ein Bremsvorgang eingeleitet wird, erhöht wird.

Zusätzlich oder alternativ wird in vorteilhafter Weise als die Aktivität eine vorbestimmte Handlung des Fahrzeuginsassen erfasst, welche einen Eingriff des Fahrzeuginsassen in das Fahrmanöver andeutet. Mit anderen Worten kann als die Aktivität eine Bewegung des Fahrzeuginsassen erfasst werden, mit welcher der Fahrzeuginsasse in das Fahrmanöver eingreifen möchte. Dabei kann als die vorbestimmte Handlung eine Fußbewegung des Fahrzeuginsassen erfasst werden, welche auf eine Betätigung eines Bremspedals oder eines Gaspedals des Kraftfahrzeugs hinweist. Alternativ oder zusätzlich kann die vorbestimmte Handlung auf eine Betätigung eines Lenkrads des Kraftfahrzeugs hinweisen. Beispielsweise kann zur Erfassung des Lenkradeingriffs die Erfassungseinrichtung eine Kamera und/oder einen Sensor aufweisen, welche oder welcher dazu eingerichtet ist, eine Annäherung zumindest eine Hand des Fahrzeuginsassen oder eine Berührung des Lenkrads durch zumindest eine Hand des Fahrzeuginsassen zu erfassen. Beispielsweise kann zur Erfassung des Pedaleingriffs an das Bremspedal oder das Gaspedal die Erfassungseinrichtung eine Kamera und/oder einen Sensor aufweisen, welche oder welcher dazu eingerichtet ist, eine Annäherung eines Fußes des Fahrzeuginsassen oder einer Berührung des jeweiligen Pedals durch den Fuß des Fahrzeuginsassen zu erfassen.

Eine vorteilhafte Weiterbildung sieht vor, dass vor dem Erfassen der Aktivität des Fahrzeuginsassen der Fahrzeuginsasse durch die Erfassungseinrichtung identifiziert wird, wobei bei der Identifizierung des Fahrzeuginsassen biometrische Merkmale erfasst werden. Hierzu kann der Fahrzeuginsasse vor dem Erfassen der zumindest eine Aktivität des Fahrzeuginsassen zunächst durch die Erfassungseinrichtung in dem Innenraum des Kraftfahrzeugs erfasst werden. Beispielsweise können als biometrische Merkmale eine Körpergröße des Fahrzeuginsassen und/oder ein Gesicht des Fahrzeuginsassen erfasst werden. Zusätzlich oder alternativ kann zur Identifizierung des Fahrzeuginsassen ein Gewicht des Fahrzeuginsassen und/oder eine Stimme des Fahrzeuginsassen erfasst werden. Zusätzlich oder alternativ kann zur Identifizierung des Fahrzeuginsassen in dem Innenraum des Kraftfahrzeugs ein Kindersitz durch die Erfassungseinrichtung erfasst werden. Beispielsweise kann ein vorbestimmtes Fahrprofil, in welchem voreingestellte Betriebsparameter der Fahrerassistenzsysteme für den Fahrzeuginsassen, hinterlegt sind, gespeichert sein. Zusätzlich oder alternativ kann es vorgesehen sein, dass nach der Erfassung oder Identifizierung des Fahrzeuginsassen eine Steuereinrichtung dazu eingerichtet ist, mit einer fahrzeugexternen Einheit zur Übermittlung von Daten, insbesondere Betriebsparameter, zu kommunizieren und diese Daten von der fahrzeugexternen Einheit zu beziehen. Damit können beispielsweise Parametersätze, welche ein Fahrverhalten des identifizierten Fahrzeuginsassen definieren oder beschreiben, auf das Kraftfahrzeug, insbesondere das zumindest eine Fahrerassistenzsystem übertragen werden. Beispielsweise können die Parametersätze ein sportliches Fahrverhalten oder ein defensives Fahrverhalten des Fahrzeuginsassen definieren oder beschreiben. Wird das Kraftfahrzeug gestartet, so können zunächst die voreingestellten, insbesondere dem Fahrzeuginsassen zugewiesenen oder zugeordneten, Parametersätze oder der zumindest eine Betriebsparameter voreingestellt sein. Durch das Erfassen der zumindest eine Reaktion des Fahrzeuginsassen auf das Fahrmanöver, welches durch das zumindest eine Fahrerassistenzsystem gesteuert oder überwacht wird, können diese Parametersätze des Fahrzeuginsassen und/oder der voreingestellte zumindest eine Betriebsparameter angepasst werden.

Eine vorteilhafte Weiterbildung der Erfindung sieht vor, dass ein Fahrzeuginsasse, der in ein Fahrzeug zum ersten Mal einsteigt und für den kein Fahrprofil oder kein Parametersatz in dem Fahrzeug gespeichert ist und/oder kein Fahrprofil oder kein Parametersatz von einer fahrzeugexternen Einheit zur Übermittlung von Daten abrufbar ist, als unbekannter und/oder neuer Fahrzeuginsasse identifiziert wird. Für den unbekannten und/oder neuen Fahrzeuginsassen wird ein neues Fahrprofil angelegt, wobei als initiale Parameterwerte generell ermittelte, voreingestellte Betriebsparameter oder Parameterwerte verwendet werden oder der unbekannte und/oder neue Fahrzeuginsasse nach gewünschten Parametern gefragt wird. Als Fahrweise kann beispielsweise eine sportliche oder defensive Fahrweise eingestellt werden und ein entsprechender Parametersatz wird für die initialen Parameterwerte geladen. Ein derartiges Vorgehen ist insbesondere dann vorteilhaft, wenn es sich bei dem unbekannten und/oder neuen Fahrzeuginsassen um den Fahrer des Fahrzeugs handelt.

Eine vorteilhafte Weiterbildung der Erfindung kann vorsehen, dass nachdem die Erfassungseinrichtung alle Fahrzeuginsassen erfasst hat und festgestellt wurde, dass für diese Konstellation der Fahrzeuginsassen noch kein Fahrprofil und/oder Parametersatz existiert, eine Abfrage erfolgt, ob eine neues Fahrprofil für den Fahrer angelegt und/oder ein bereits bestehendes Fahrprofil des Fahrers um die neue Konstellation der Fahrzeuginsassen ergänzt werden soll. Eine Abfrage bezüglich der Anlage eines neuen Fahrprofils für den Fahrer und/oder die Ergänzung eines bereits bestehenden Fahrprofils hat den Vorteil, dass auf Veränderungen in der Konstellation der Fahrzeuginsassen reagiert werden kann.

Vorteilhafterweise könnte auch die Position der Fahrzeuginsassen im Fahrzeug berücksichtigt werden. Insbesondere ob sich ein Fahrzeuginsasse auf dem Beifahrersitz oder auf einem Rücksitz befindet. So wird ein Fahrzeuginsassen, insbesondere ein Kind, auf einem Rücksitz unter Umständen schneller Reisekrank, als wenn der Fahrzeuginsasse auf dem Beifahrersitz sitzen würde. Folglich müsste bei einer Position des Fahrzeuginsassen, insbesondere des Kindes, auf einem Rücksitz, eine defensivere Fahrweise angewandt werden, als wenn der Fahrzeuginsasse, insbesondere das Kind, auf dem Beifahrersitz sitzen würde.

Gemäß einer vorteilhaften Weiterbildung werden durch die Erfassungseinrichtung zusätzlich Daten einer Vorrichtung, welche eine Terminvorgabe und/oder persönliche Kalenderdaten umfassen, erfasst, welche bei der Anpassung des zumindest einen Betriebsparameters berücksichtigt werden. Bei der "Vorrichtung" handelt es sich bevorzugt um eine fahrzeugexterne Einheit, insbesondere eine Servereinrichtung im Internet oder portables mobiles Endgerät. Mit anderen Worten können weitere Daten oder Datensätze bei der Anpassung des zumindest einen Betriebsparameters berücksichtigt werden. Bei den weiteren Daten kann es sich zum Beispiel um Daten eines Terminkalenders handeln, welche auf einem mit dem Kraftfahrzeug gekoppelten portablen mobilen Endgerät hinterlegt sind. Beispielsweise kann die Erfassungseinrichtung durch einen Abgleich oder Erfassen der weiteren Daten erkennen, ob der Fahrzeuginsasse einen Termin schnell erreichen muss und somit eine schnellere Fahrweise erwartet.

In vorteilhafter Weise wird das Kraftfahrzeug während dem Fahrmanöver manuell oder teilautonom oder autonom, insbesondere vollautomatisiert, betrieben. Mit autonomen ist hier bevorzugt gemeint, dass sich das Kraftfahrzeug selbstständig bewegt. Im autonomen Fahrbetrieb kann es vorgesehen sein, dass sich keiner im Kraftfahrzeug oder kein aktiver Fahrzeugführer oder nur Passagiere im Kraftfahrzeug befinden. Beispielsweise wird im autonomen Fahrbetrieb das Kraftfahrzeug durch das zumindest eine Fahrerassistenzsystem oder mehrere Fahrerassistenzsysteme gesteuert. Im autonomen Fahrbetrieb muss sich der Fahrer des Kraftfahrzeugs also nicht weiter um den weiteren Betrieb des Kraftfahrzeugs kümmern. Beispielsweise kann das Kraftfahrzeug autonom eine Kurvenfahrt durchführen. Mit manuell ist hier bevorzugt gemeint, dass das Kraftfahrzeug durch einen Fahrer gesteuert wird. Mit teilautonom ist hier bevorzugt gemeint, dass das Kraftfahrzeug für vorbestimmte Fahrmanöver, wie beispielsweise Einparken, autonom durchführt und für einen Fahrbetrieb durch den Fahrer gesteuert wird.

Zu der Erfindung gehört auch ein System zum Anpassen zumindest eines Betriebsparameters des Kraftfahrzeugs während eines Fahrmanövers des Kraftfahrzeugs. Das System umfasst zumindest ein Fahrerassistenzsystem, welches dazu eingerichtet ist, den zumindest einen Betriebsparameter einzustellen. Ferner weist das System eine Erfassungseinrichtung auf, welche dazu eingerichtet ist, zumindest eine Aktivität eines Fahrzeuginsassen während des Fahrmanövers in Reaktion auf das Fahrmanöver zu erfassen. Schließlich umfasst das System eine Steuereinrichtung, welche dazu eingerichtet ist, den zumindest einen Betriebsparameter des Kraftfahrzeugs in Abhängigkeit von der erfassten Aktivität des Fahrzeuginsassen durch Ansteuern des zumindest einen Fahrerassistenzsystems anzupassen. Bei dem Fahrerassistenzsystem kann es sich beispielsweise um einen Abstands-Regel-Tempomat und/oder um ein Spurhalteassistenten und/oder um einen Spurwechselassistent und/oder um eine Einparkhilfe und/oder um einen Notbremsassistent und/oder um eine intelligente Geschwindigkeitsassistenz und/oder um einen Beschleunigungs-Assistent und/oder um ein Antiblockiersystem (kurz ABS) und/oder um ein Elektronisches Stabilitätsprogramm (kurz ESP) handeln. Zusätzlich oder alternativ kann die Erfassungseinrichtung dazu eingerichtet sein, die auf das Fahrmanöver hin erfassten Daten des zumindest einen Fahrzeuginsassen zu speichern oder zu sammeln und aus den Daten ein statistisches Muster im Verhalten oder in der Aktivität des Fahrzeuginsassen zu bestimmen und/oder zu speichern. Die Erfassungseinrichtung ist ferner dazu eingerichtet, eine Bestätigungshandlung auf eine Zustandsanfrage hinsichtlich der Reaktion des Fahrzeuginsassen auf das Fahrmanöver, welche durch eine Ausgabeeinrichtung ausgegeben wird, zu erfassen, wobei die Steuereinrichtung dazu eingerichtet ist, auf die Bestätigungshandlung hin den zumindest einen Betriebsparameter des Kraftfahrzeugs anzupassen. Die Erfassungseinrichtung ist ferner dazu eingerichtet, als Aktivität eine Schutzhandlung wenigstens eines Fahrzeuginsassen zum Personenschutz des Fahrzeuginsassen zu erfassen, wobei als Schutzhandlung eine akustische Äußerung des Fahrzeuginsassen und/oder ein Festhalten und/oder Abstützen des Fahrzeuginsassen an einem Innenausstattungselement des Kraftfahrzeugs erfasst wird.

Zu der Erfindung gehört auch ein Kraftfahrzeug mit einem erfindungsgemäßen System. Das Kraftfahrzeug ist bevorzugt als Kraftwagen, insbesondere als Personenkraftwagen, ausgebildet.

Zu der Erfindung gehören auch Weiterbildungen des erfindungsgemäßen Systems und des erfindungsgemäßen Kraftfahrzeugs, die Merkmale aufweisen, wie sie bereits im Zusammenhang mit den Weiterbildungen des erfindungsgemäßen Verfahrens beschrieben worden sind. Aus diesem Grund sind die entsprechenden Weiterbildungen des erfindungsgemäßen Systems und des erfindungsgemäßen Kraftfahrzeugs hier nicht noch einmal beschrieben.

Im Folgenden ist ein Ausführungsbeispiel der Erfindung beschrieben. Hierzu zeigt die einzige Figur (Fig.) in einer schematischen Darstellung die einzelnen Verfahrensschritte des Verfahrens zum Anpassen zumindest eines Betriebsparameters eines Kraftfahrzeugs in einem Ablaufdiagramm.

Bei dem im Folgenden erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren ist die beschriebene Ausführungsform auch durch weitere der bereits beschriebenen Merkmale der Erfindung ergänzbar.

In einem ersten Verfahrensschritt S1 wird mittels einer Erfassungseinrichtung 10 eines Systems zum Anpassen zumindest eines Betriebsparameters eines Kraftfahrzeugs 16 ein Fahrzeuginsasse 12 in einem Innenraum 14 des Kraftfahrzeugs 16 erfasst. Bei dem Fahrzeuginsassen 12 kann es sich beispielsweise um einen Fahrer oder Mitfahrer des Kraftfahrzeugs 16 handeln. Beim Erfassen des Fahrzeuginsassen 12 kann die Erfassungseinrichtung 10 zusätzlich oder alternativ dazu eingerichtet sein, den erfassten Fahrzeuginsassen 12 zu identifizieren. Zum Identifizieren des Fahrzeuginsassen 12 kann die Erfassungseinrichtung 10 beispielsweise dazu eingerichtet sein, biometrische Merkmale, wie beispielsweise eine Körpergröße und/oder ein Gesicht, und/oder eine Stimme und/oder ein Gewicht des Fahrzeuginsassen 12 zu erfassen. Zum Erfassen des Gesichts und/oder der Körpergröße des Fahrzeuginsassen 12 kann die Erfassungseinrichtung 10 beispielsweise eine Kamera aufweisen. Zum Erfassen des Gewichts des Fahrzeuginsassen 12 kann die Erfassungseinrichtung 10 beispielsweise einen Gewichtssensor aufweisen. Zum Erfassen der Stimme des Fahrzeuginsassen 12 kann die Erfassungseinrichtung 10 beispielsweise ein Mikrofon aufweisen, welches dazu eingerichtet ist, eine Stimmerkennung durchzuführen. Zum Identifizieren des Fahrzeuginsassen kann die Steuereinrichtung dazu eingerichtet sein, die erfassten Daten des Fahrzeuginsassen in der Steuereinrichtung hinterlegten Daten zu vergleichen. Zudem kann die Steuereinrichtung dazu eingerichtet sein, die Daten einem Fahrzeuginsassen zuzuordnen. Sobald der Fahrzeuginsasse 12 durch die Erfassungseinrichtung 10 erfasst und/oder identifiziert wurde, kann die Steuereinrichtung dazu eingerichtet sein, für den Fahrzeuginsassen 12 voreingestellte Fahrzeugparameter und/oder Betriebsparameter vorzugeben oder von einer fahrzeugexternen Einheit abzufragen. Mit anderen Worten können spezifisch für den erfassten Fahrzeuginsassen zumindest einem Fahrerassistenzsystem oder mehreren Fahrerassistenzsystemen des Kraftfahrzeugs 16 voreingestellte Fahrzeugparameter und/oder Betriebsparameter zugewiesen werden. Fährt das Kraftfahrzeug 16 los oder wird in Betrieb genommen, so wird das Kraftfahrzeug 16 oder wird ein Fahrmanöver des Kraftfahrzeugs 16 in Abhängigkeit der voreingestellten Parameter oder Parametersätze gesteuert.

Im Betrieb des Kraftfahrzeugs 16 kann der zumindest eine Betriebsparameter oder die Fahrzeugparameter des Kraftfahrzeugs 16 voreingestellt sein. Beispielsweise kann dem identifizierten Fahrzeuginsassen 12 ein Fahrprofil zugeordnet sein. Dem Fahrprofil kann der zumindest eine Betriebsparameter des Kraftfahrzeugs 16 oder mehrere Betriebsparameter des Kraftfahrzeugs 16 hinterlegt sein. Das Fahrprofil oder mehrere Fahrprofile, welche jeweils einem Fahrzeuginsassen zugewiesen sein können, können beispielsweise in einem Speicher der Steuereinrichtung (in Fig. nicht gezeigt) gespeichert sein. Die Steuereinrichtung kann dazu eingerichtet sein, den erfassten Fahrzeuginsassen 12 dem Fahrprofil zuzuordnen. Durch das bestimmte Fahrprofil kann der zumindest eine Betriebsparameter des Kraftfahrzeugs 16 oder mehrere Betriebsparameter des Kraftfahrzeugs 16 voreingestellt sein. Beispielsweise kann für den erfassten Fahrzeuginsassen 12 der vorbestimmte Abstand des Abstands-Regel-Tempomat den hinterlegt sein.

Das Kraftfahrzeug 16 ist bevorzugt als Kraftwagen, insbesondere Personenkraftwagen ausgebildet. Ferner weist das Kraftfahrzeug 16 zumindest ein Fahrerassistenzsystem auf. Bevorzugt weist das Kraftfahrzeug mehrere Fahrerassistenzsysteme auf. Das zumindest eine Fahrerassistenzsystem ist dazu eingerichtet, ein Fahrmanöver des Kraftfahrzeugs 16 durchzuführen oder zu steuern oder zu überwachen. Mittels des zumindest einen Fahrerassistenzsystems des Kraftfahrzeugs 16 wird ein Betriebsparameter des Kraftfahrzeugs 16 während des Fahrmanövers eingestellt. Mit anderen Worten kann zum Durchführen des Fahrmanövers das Fahrerassistenzsystem des Kraftfahrzeugs 16 zumindest ein Betriebsparameter des Kraftfahrzeugs 16 eingestellt oder vorgegeben werden.

Beispielsweise kann es sich bei dem Fahrmanöver um ein Fahren des Kraftfahrzeugs 16 und/oder ein Überholen und/oder einen Spurwechsel und/oder ein Abbiegen und/oder ein Rückwärtsfahren und/oder ein Anhalten und/oder ein Anfahren und/oder ein Beschleunigen und/oder eine Kurvenfahrt und/oder ein Einparken und/oder ein Ausparken des Kraftfahrzeugs 16 handeln. Zum Durchführen des jeweiligen Fahrmanövers ist das zumindest eine Fahrerassistenzsystem oder sind mehrere Fahrerassistenzsysteme zuständig. Hierzu stellt das jeweilige Fahrerassistenzsystem zumindest einen Betriebsparameter des Kraftfahrzeugs 16 ein. Bei dem zumindest einen Betriebsparameter kann es sich um eine Geschwindigkeit des Kraftfahrzeugs und/oder eine Beschleunigung, insbesondere eine Querbeschleunigung des Kraftfahrzeugs und/oder eine Längsbeschleunigung des Kraftfahrzeugs, und/oder ein Abstand zu einem vorausfahrehrenden Kraftfahrzeug und/oder ein Halten einer Fahrspur des Kraftfahrzeugs und/oder ein Lenkmoment handeln.

Beispielsweise kann zum Durchführen einer Kurvenfahrt des Kraftfahrzeugs 16 als Fahrmanöver das zumindest eine Fahrerassistenzsystem ein Lenkmoment und/oder eine Querbeschleunigung als zumindest einen Betriebsparameter vorgeben. Handelt es sich bei dem zumindest einem Fahrerassistenzsystem beispielsweise um einen Abstands-Regel-Tempomat, so ist das Fahrerassistenzsystem dazu eingerichtet, als Fahrmanöver bei einer vorbestimmten Geschwindigkeit des Kraftfahrzeugs 16 einen vorbestimmten Abstand zu einem vorausfahrenden Kraftfahrzeug einzuhalten und/oder bei Unterschreiten des vorbestimmten Abstands zu dem vorausfahrenden Kraftfahrzeug einen Bremsvorgang einzuleiten. Als Betriebsparameter kann bei dem Abstands-Regel-Tempomat der vorbestimmten Abstand zu einem vorausfahrenden Fahrzeug und/oder eine Geschwindigkeit des Kraftfahrzeugs 16 vorgegeben sein.

In einem zweiten Verfahrensschritt S2 wird eine Aktivität des Fahrzeuginsassen 12 während des Fahrmanövers in Reaktion auf das Fahrmanöver mittels der Erfassungseinrichtung 10 erfasst. Mit anderen Worten kann die Erfassungseinrichtung 10 dazu eingerichtet sein, eine Veränderung eines Zustands des Fahrzeuginsassen 12 zu erfassen. Hierbei kann die Erfassungseinrichtung 10 dazu eingerichtet sein, ein bewusstes Verhalten und/oder unbewusst das Verhalten des Fahrzeuginsassen 12 zu erkennen. Durch das Erkennen des bewussten und/oder unbewussten Verhaltens des Fahrzeuginsassen 12 können Rückschlüsse auf das aktuelle Fahrmanöver geschlossen werden. Als Aktivität des Fahrzeuginsassen 12 kann die Erfassungseinrichtung 10 dazu eingerichtet sein, eine Handlung H des Fahrzeuginsassen 12 und/oder einen Zustandsparameters Z des Fahrzeuginsassen 12 zu erfassen. Beispielsweise kann die Erfassungseinrichtung 10 dazu eingerichtet sein, während einer Beschleunigung oder einer Kurvenfahrt des Kraftfahrzeugs 16 als das aktuelle Fahrmanöver beispielsweise eine Annäherung eines Fußes des Fahrzeuginsassen an ein Bremspedal oder ein Festhalten an einem Griff des Kraftfahrzeugs 16 als Handlung des Fahrzeuginsassen 12 zu erfassen.

Durch das Erfassen der Handlung H des Fahrzeuginsassen 12 kann eine Steuereinrichtung in einem dritten Verfahrensschritt S3 den zumindest einen Betriebsparameter des Kraftfahrzeugs 16 in Abhängigkeit von der erfassten Aktivität des Fahrzeuginsassen 12 durch Ansteuern des zumindest einen Fahrerassistenzsystems anpassen. Gibt das zumindest eine Fahrerassistenzsystem während der Kurvenfahrt des Kraftfahrzeugs 16 als das aktuelle Fahrmanöver des Kraftfahrzeugs 16 eine Beschleunigung als den zumindest einen Betriebsparameter vor, so kann die Steuereinrichtung durch Erfassen der Handlung H beim Anpassen des zumindest einen Betriebsparameters beispielsweise die Beschleunigung oder Geschwindigkeit reduzieren. Erfasst beispielsweise die Erfassungseinrichtung 10 während der Kurvenfahrt als Fahrmanöver als Handlung H des Fahrzeuginsassen 12 eine Annäherung eines Fußes an ein Gaspedals des Kraftfahrzeugs 16, so kann die Steuereinrichtung beim Anpassen des zumindest einen Betriebsparameters beispielsweise die Beschleunigung erhöhen.

Im Folgenden sind noch vier konkrete Ausführungsbeispiele beschrieben.

In dem ersten Ausführungsbeispiel steigt ein Fahrer als Fahrzeuginsasse 12 des Kraftfahrzeugs 16 zum ersten Mal in das Kraftfahrzeug 16 ein. Die Erfassungseinrichtung erkennt in dem Innenraum 14 des Kraftfahrzeugs 16 biometrische Daten oder andere Daten des Fahrers. Zusätzlich oder alternativ kann die Erfassungseinrichtung 10 dazu eingerichtet sein, die erfassten biometrischen Daten oder anderen Daten mit weiteren Daten, welche in einer fahrzeugexternen Einheit gespeichert sind, abzugleichen. Die Erfassungseinrichtung 10 kann in diesem Ausführungsbeispiel feststellen, dass für den Fahrzeuginsassen 12 noch keine Daten zu einer gewünschten Fahrweise des Fahrzeuginsassen 12 vorhanden sind. Somit können wahlweise generell ermittelte, voreingestellte Betriebsparameter oder Parameterwerte verwendet werden, oder der Fahrer nach den gewünschten Parametern gefragt werden. Als Fahrweise kann beispielsweise eine sportliche oder defensive Fahrweise eingestellt werden. In einer ersten Fahrsituation erkennt die Erfassungseinrichtung 10, dass der Fahrer selbst etwas früher gebremst hätte, beispielsweise durch reflexartiges Greifen des Fahrers an einen Türgriff, als ein zweites Kraftfahrzeug vor dem eigenen einschert und dadurch eine Bremsung einleitet. Daraufhin wird in dem Fahrerassistenzsystem der Abstandsparameter als der Betriebsparameter geändert, um bei einer erneuten, gleichartigen Situation, dem Fahrer ein besseres Gefühl zu vermitteln. Diese Parameteränderung wird gegebenenfalls mit der fahrzeugexternen Zentrale oder Einrichtung kommuniziert, um für andere Erstfahrer des Kraftfahrzeugs 16 die Parameter ebenfalls anzupassen.

In dem zweiten Ausführungsbeispiel steigt der Fahrer als Fahrzeuginsasse 12 in das Kraftfahrzeug 16. Durch die Erfassungseinrichtung 10, beispielsweise Sensoren, wird festgestellt, dass der Herzschlag des Fahrers erhöht ist. Zudem wird durch die Erfassungseinrichtung erfasst, dass im Terminkalender ein wichtiger Termin gespeichert ist, der nur knapp erreicht wird. Das System geht davon aus, dass der Fahrer es eilig hat und setzt somit für diese Fahrt die maximale Querbeschleunigung als Betriebsparameter für die Kurvenfahrt hoch.

In dem dritten Ausführungsbeispiel steigt der Fahrer in das Kraftfahrzeug 16 und setzt einen Kindersitz auf die Rückbank des Kraftfahrzeugs 16, was durch die Erfassungseinrichtung 10 erkannt wird. Die Steuereinrichtung des Systems ist daraufhin bevorzugt dazu eingerichtet, eigenständig eine defensive Fahrweise, also eine defensivere Parameterwahl, vorzugeben oder einzustellen.

In dem vierten Ausführungsbeispiel wird durch die Erfassungseinrichtung 10 erkannt, dass der Fahrer auf dem morgendlichen Weg zur Arbeit schneller fahren möchte, als auf dem abendlichen Rückweg. Die Steuereinrichtung passt daraufhin die Parameter des Fahrerassistenzsystems oder der mehreren Fahrerassistenzsysteme an diesen tageszeitlichen Rhythmus an.

Insgesamt zeigt das Beispiel, wie durch die Erfindung eine automatische Erkennung der Passagier-Komfortzone erfolgt.

## Patentansprüche

1. Verfahren zum Anpassen zumindest eines Betriebsparameters eines Kraftfahrzeugs (16) während eines Fahrmanövers des Kraftfahrzeugs (16), wobei der zumindest eine Betriebsparameter des Kraftfahrzeugs (16) durch zumindest ein Fahrerassistenzsystem des Kraftfahrzeugs eingestellt (16) wird, umfassend die Schritte:
- Identifizieren wenigstens eines Fahrzeuginsassen, wobei bei der Identifizierung des Fahrzeuginsassen biometrische Merkmale erfasst werden,
- Erstellen oder Laden eines vorbestimmten Fahrprofils für den Fahrzeuginsassen,
- Erfassen zumindest einer Aktivität eines Fahrzeuginsassen (12) während des Fahrmanövers in Reaktion auf das Fahrmanöver mittels einer Erfassungseinrichtung (10) und
- Anpassen des zumindest einen Betriebsparameters des Kraftfahrzeugs (16) in Abhängigkeit von der erfassten Aktivität des Fahrzeuginsassen (12) durch Ansteuern des zumindest einen Fahrerassistenzsystems mittels einer Steuereinrichtung,
- Speichern des wenigstens einen angepassten Betriebsparameters in dem Fahrprofil des Fahrzeuginsassen
**dadurch gekennzeichnet, dass**
zum Anpassen des zumindest einen Betriebsparameters des Kraftfahrzeugs (16) eine Bestätigungshandlung auf eine Zustandsanfrage hinsichtlich der Reaktion des Fahrzeuginsassen (12) auf das Fahrmanöver, welche durch eine Ausgabeeinrichtung ausgegeben wird, erfasst wird, wobei als Aktivität eine Schutzhandlung wenigstens eines Fahrzeuginsassen (12) zum Personenschutz des Fahrzeuginsassen (12) erfasst wird, wobei als Schutzhandlung eine akustische Äußerung des Fahrzeuginsassen (12) und/oder ein Festhalten und/oder Abstützen des Fahrzeuginsassen (12) an einem Innenausstattungselement des Kraftfahrzeugs (16) erfasst wird.

2. Verfahren nach Anspruch 1,
**dadurch gekennzeichnet, dass**
als die Aktivität eine Herzfrequenz und/oder eine Atemfrequenz und/oder eine Gehirnaktivität und/oder ein Müdigkeitszustand wenigstens eines Fahrzeuginsassen (12) erfasst wird.

3. Verfahren nach Anspruch 1 oder 2,
**dadurch gekennzeichnet, dass**
als die Aktivität eine vorbestimmte Handlung wenigstens eines Fahrzeuginsassen (12) erfasst wird, welche einen Eingriff des Fahrzeuginsassen (12) in das Fahrmanöver andeutet, wobei als die vorbestimmte Handlung (H) eine Fußbewegung des Fahrzeuginsassen (12) erfasst wird, welche auf ein Betätigen eines Bremspedals oder eines Gaspedals des Kraftfahrzeugs (16) hinweist, oder die vorbestimmte Handlung (H) auf eine Betätigung eines Lenkrads des Kraftfahrzeugs (16) hinweist.

4. Verfahren nach einem der vorhergehenden Ansprüche,
**dadurch gekennzeichnet, dass**
als der zumindest eine Betriebsparameter eine Geschwindigkeit und/oder eine Querbeschleunigung und/oder eine Längsbeschleunigung und/oder ein Abstand zu einem vorausfahrehrenden Kraftfahrzeug und/oder ein Halten einer Fahrspur des Kraftfahrzeugs (16) und/oder ein Lenkmoment angepasst wird.

5. Verfahren nach einem der vorhergehenden Ansprüche,
**dadurch gekennzeichnet, dass**
bei der Identifizierung des Fahrzeuginsassen (12) mittels biometrischer Merkmale eine Körpergröße und/oder ein Gesicht, und/oder ein Gewicht und/oder eine Stimme des Fahrzeuginsassen (12) erfasst wird.

6. Verfahren nach einem der vorhergehenden Ansprüche,
**dadurch gekennzeichnet, dass**
durch die Erfassungseinrichtung (10) zusätzlich Daten einer Vorrichtung erfasst werden, welche eine Terminvorgabe und/oder persönliche Kalenderdaten umfassen, erfasst werden, welche bei der Anpassung des zumindest eines Betriebsparameters berücksichtigt werden.

7. System zum Anpassen zumindest eines Betriebsparameters eines Kraftfahrzeugs (16) während eines Fahrmanövers des Kraftfahrzeugs (16) umfassend:
- zumindest ein Fahrerassistenzsystem, welches dazu eingerichtet ist, den zumindest einen Betriebsparameter einzustellen,
- eine Erfassungseinrichtung (10), welche dazu eingerichtet ist, zumindest eine Aktivität eines Fahrzeuginsassen (12) während des Fahrmanövers in Reaktion auf das Fahrmanöver zu erfassen und
- eine Steuereinrichtung, welche dazu eingerichtet ist, den zumindest einen Betriebsparameters des Kraftfahrzeugs (16) in Abhängigkeit von der erfassten Aktivität des Fahrzeuginsassen (12) durch Ansteuern des zumindest einen Fahrerassistenzsystems anzupassen,
**dadurch gekennzeichnet, dass**
die Erfassungseinrichtung ferner dazu eingerichtet ist, eine Bestätigungshandlung auf eine Zustandsanfrage hinsichtlich der Reaktion des Fahrzeuginsassen (12) auf das Fahrmanöver, welche durch eine Ausgabeeinrichtung ausgegeben wird, zu erfassen, wobei die Steuereinrichtung dazu eingerichtet ist, auf die Bestätigungshandlung hin den zumindest einen Betriebsparameter des Kraftfahrzeugs (16) anzupassen, wobei die Erfassungseinrichtung (10) ferner dazu eingerichtet ist, als Aktivität eine Schutzhandlung wenigstens eines Fahrzeuginsassen (12) zum Personenschutz des Fahrzeuginsassen (12) zu erfassen, wobei als Schutzhandlung eine akustische Äußerung des Fahrzeuginsassen (12) und/oder ein Festhalten und/oder Abstützen des Fahrzeuginsassen (12) an einem Innenausstattungselement des Kraftfahrzeugs (16) erfasst wird.

8. System nach Anspruch 7,
**dadurch gekennzeichnet, dass**
die Erfassungseinrichtung (10) eine Kamera und/oder einen Sensor, insbesondere einen kapazitiven Sensor, umfasst.

9. Kraftfahrzeug (16) mit einem System nach einem der Ansprüche 7 oder 8.

## Claims

1. Method for adapting at least one operating parameter of a motor vehicle (16) during a driving manoeuvre of the motor vehicle (16), the at least one operating parameter of the motor vehicle (16) being set by at least one driver assistance system of the motor vehicle (16), comprising the steps of:
- identifying at least one vehicle occupant, biometric features being detected for identifying the vehicle occupant,
- creating or loading a predetermined driving profile for the vehicle occupant,
- detecting at least one activity by a vehicle occupant (12) during the driving manoeuvre in reaction to the driving manoeuvre by means of a detection device (10), and
- adapting the at least one operating parameter of the motor vehicle (16) on the basis of the detected activity by the vehicle occupant (12) by actuating the at least one driver assistance system by means of a control device,
- storing the at least one adapted operating parameter in the driving profile of the vehicle occupant,
**characterized in that**
the at least one operating parameter of the motor vehicle (16) is adapted by detecting a confirmation action for a status request regarding the reaction by the vehicle occupant (12) to the driving manoeuvre, which request is output by an output device, wherein the detected activity is a protective action by at least one vehicle occupant (12) to protect the person of the vehicle occupant (12), the protective action detected being an audible utterance by the vehicle occupant (12) and/or the vehicle occupant's (12) holding onto and/or bracing against an interior fixture element of the motor vehicle (16).

2. Method according to Claim 1,
**characterized in that**
the detected activity is the heart rate and/or the respiratory rate and/or the brain activity and/or the drowsiness of at least one vehicle occupant (12).

3. Method according to Claim 1 or 2,
**characterized in that**
the detected activity is a predetermined action by at least one vehicle occupant (12) that indicates an intervention by the vehicle occupant (12) in the driving manoeuvre, the predetermined action (H) detected being a foot movement by the vehicle occupant (12) that indicates operation of a brake pedal or an accelerator pedal of the motor vehicle (16), or the predetermined action (H) indicating operation of a steering wheel of the motor vehicle (16).

4. Method according to one of the preceding claims, **characterized in that**
the at least one operating parameter adapted is a velocity and/or a lateral acceleration and/or a longitudinal acceleration and/or a distance from a motor vehicle driving ahead and/or the keeping of the motor vehicle (16) in a lane and/or a steering torque.

5. Method according to one of the preceding claims, **characterized in that**
the body size and/or face and/or weight and/or voice of the vehicle occupant (12) is detected for identifying the vehicle occupant (12) by means of biometric features.

6. Method according to one of the preceding claims, **characterized in that**
the detection device (10) additionally acquires data from an apparatus that include a specified appointment and/or personal calendar data, which are taken into consideration for adapting the at least one operating parameter.

7. System for adapting at least one operating parameter of a motor vehicle (16) during a driving manoeuvre of the motor vehicle (16), comprising:
- at least one driver assistance system, which is configured to set the at least one operating parameter,
- a detection device (10), which is configured to detect at least one activity by a vehicle occupant (12) during the driving manoeuvre in reaction to the driving manoeuvre, and
- a control device, which is configured to adapt the at least one operating parameter of the motor vehicle (16) on the basis of the detected activity by the vehicle occupant (12) by actuating the at least one driver assistance system,
**characterized in that**
the detection device is furthermore configured to detect a confirmation action for a status request regarding the reaction by the vehicle occupant (12) to the driving manoeuvre, which request is output by an output device, wherein the control device is configured to respond to the confirmation action by adapting the at least one operating parameter of the motor vehicle (16), wherein the detection device (10) is furthermore configured to detect a protective action by at least one vehicle occupant (12) to protect the person of the vehicle occupant (12) as the activity, the protective action detected being an audible utterance by the vehicle occupant (12) and/or the vehicle occupant's (12) holding onto and/or bracing against an interior fixture element of the motor vehicle (16).

8. System according to Claim 7,
**characterized in that**
the detection device (10) comprises a camera and/or a sensor, in particular a capacitive sensor.

9. Motor vehicle (16) having a system according to either of Claims 7 and 8.

## Revendications

1. Procédé pour adapter au moins un paramètre de fonctionnement d'un véhicule automobile (16) pendant la conduite du véhicule automobile (16), ledit au moins un paramètre de fonctionnement du véhicule automobile (16) étant réglé (16) par au moins un système d'aide à la conduite du véhicule automobile, comprenant les étapes suivantes :
- identification d'au moins un occupant du véhicule, des caractéristiques biométriques étant détectées lors de l'identification de l'occupant du véhicule,
- création ou chargement d'un profil de conduite prédéterminé pour l'occupant du véhicule,
- détection d'au moins une activité d'un occupant du véhicule (12) pendant la conduite en réaction à la manoeuvre de conduite, au moyen d'un dispositif de détection (10), et
- adaptation dudit au moins un paramètre de fonctionnement du véhicule automobile (16) en fonction de l'activité détectée de l'occupant du véhicule (12) en activant ledit au moins un système d'aide à la conduite au moyen d'un dispositif de commande,
- enregistrement dudit au moins un paramètre de fonctionnement adapté dans le profil de conduite de l'occupant du véhicule,
**caractérisé en ce que**
pour adapter ledit au moins un paramètre de fonctionnement du véhicule automobile (16), une action de confirmation est enregistrée en réponse à une demande d'état concernant la réaction de l'occupant du véhicule (12) à la manoeuvre de conduite, laquelle est émise par un dispositif de sortie, une action de protection d'au moins un occupant du véhicule (12) pour la protection personnelle de l'occupant du véhicule (12) étant détectée en tant qu'activité, une expression acoustique de l'occupant du véhicule (12) et/ou un maintien et/ou un appui de l'occupant du véhicule (12) sur un élément d'équipement intérieur du véhicule automobile (16) étant détectés en tant qu'action de protection.

2. Procédé selon la revendication 1,
**caractérisé en ce que**
une fréquence cardiaque et/ou une fréquence respiratoire et/ou une activité cérébrale et/ou un état de fatigue d'au moins un occupant (12) du véhicule est détectée en tant que ladite activité.

3. Procédé selon la revendication 1 ou 2,
**caractérisé en ce que**,
lorsque l'activité est détectée comme étant une action prédéterminée d'au moins un occupant (12) du véhicule, qui indique une intervention de l'occupant (12) du véhicule dans la manoeuvre de conduite, l'action prédéterminée (H) est un mouvement du pied de l'occupant (12) du véhicule, qui se fait en réponse à un actionnement d'une pédale de frein ou d'une pédale d'accélérateur du véhicule automobile (16), ou l'action prédéterminée (H) indique un actionnement du volant du véhicule automobile (16) .

4. Procédé selon l'une des revendications précédentes, **caractérisé en ce que**
en tant qu'au moins un paramètre de fonctionnement, sont adaptés une vitesse et/ou une accélération transversale et/ou une accélération longitudinale et/ou une distance par rapport à un véhicule automobile qui précède et/ou un maintien d'une trajectoire du véhicule automobile (16) et/ou un couple de direction.

5. Procédé selon l'une des revendications précédentes, **caractérisé en ce que**
lors de l'identification de l'occupant (12) du véhicule au moyen de caractéristiques biométriques, une taille et/ou un visage, et/ou un poids et/ou une voix de l'occupant (12) du véhicule sont détectés.

6. Procédé selon l'une des revendications précédentes, **caractérisé en ce que**
le dispositif de détection (10) détecte en outre des données provenant d'un dispositif, qui comprennent une prescription de rendez-vous et/ou des données de calendrier personnel, qui sont prises en compte lors de l'adaptation d'au moins un paramètre de fonctionnement.

7. Système d'adaptation d'au moins un paramètre de fonctionnement d'un véhicule automobile (16) pendant une manoeuvre de conduite du véhicule automobile (16) comprenant :
- au moins un système d'aide à la conduite, qui est conçu pour ajuster ledit au moins un paramètre de fonctionnement,
- un dispositif de détection (10) qui est conçu pour détecter au moins une activité d'un occupant du véhicule (12) pendant la conduite en réaction à une manoeuvre de conduite, et
- un dispositif de commande, qui est conçu pour adapter ledit au moins un paramètre de fonctionnement du véhicule automobile (16) en fonction de l'activité détectée de l'occupant du véhicule (12) en commandant ledit au moins un système d'aide à la conduite,
**caractérisé en ce que**
le dispositif de détection est en outre conçu pour détecter une action de confirmation sur une demande d'état concernant la réaction de l'occupant du véhicule (12) à la manoeuvre de conduite, laquelle est émise par un dispositif de sortie, le dispositif de commande étant conçu pour adapter ledit au moins un paramètre de fonctionnement du véhicule automobile (16) en réponse à l'action de confirmation, le dispositif de détection (10) étant en outre agencé pour détecter, en tant qu'activité, une action de protection d'au moins un occupant (12) du véhicule pour la protection personnelle de l'occupant (12) du véhicule, l'action de protection détectée étant une expression acoustique de l'occupant (12) du véhicule et/ou un maintien et/ou un appui de l'occupant (12) du véhicule sur un élément d'équipement intérieur du véhicule automobile (16).

8. Système selon la revendication 7,
**caractérisé en ce que**
le dispositif de détection (10) comprend une caméra et/ou un capteur, notamment un capteur capacitif.

9. Véhicule automobile (16) comportant un système selon l'une des revendications 7 ou 8.
